Europäisches Patentamt

European Patent Office

Office européen des brevets

(19)

(11) **EP 1 299 090 B1**

(12) **FASCICULE DE BREVET EUROPEEN**

(45) Date de publication et mention
de la délivrance du brevet:
**08.03.2006 Bulletin 2006/10**

(51) Int Cl.:
***A61K 9/20*** (2006.01)   ***A61K 9/50*** (2006.01)
***A61K 9/62*** (2006.01)

(21) Numéro de dépôt: **01955397.3**

(22) Date de dépôt: **10.07.2001**

(86) Numéro de dépôt international:
**PCT/FR2001/002224**

(87) Numéro de publication internationale:
**WO 2002/003964 (17.01.2002 Gazette 2002/03)**

(54) **COMPOSITION PHARMACEUTIQUE ORALE A LIBERATION CONTROLEE ET A ABSORPTION PROLONGEE**

PHARMAZEUTISCHE ZUSAMMENSETZUNG MIT GESTEUERTER FREIGABE UND VERZÖGERTER ABSORPTION

ORAL PHARMACEUTICAL COMPOSITION WITH CONTROLLED RELEASE AND PROLONGED ABSORPTION

(84) Etats contractants désignés:
**AT BE CH CY DE DK ES FI FR GB GR IE IT LI LU MC NL PT SE TR**

(30) Priorité: **11.07.2000 FR 0009047**

(43) Date de publication de la demande:
**09.04.2003 Bulletin 2003/15**

(73) Titulaire: **FLAMEL TECHNOLOGIES**
**69693 Venissieux Cédex (FR)**

(72) Inventeurs:
• **CASTAN, Catherine**
**F-69530 Orlienas (FR)**

• **LEGRAND, Valérie**
**F-91230 Montgeron (FR)**
• **MEYRUEIX, Rémi**
**F-69009 Lyon (FR)**
• **SOULA, Gérard**
**F-69330 Meyzieu (FR)**

(74) Mandataire: **Fleurance, Raphael et al**
**Cabinet Plasseraud**
**65/67 rue de la Victoire**
**75440 Paris Cedex 09 (FR)**

(56) Documents cités:
**EP-A- 0 624 371          EP-A- 0 709 087**
**US-A- 5 268 182**

EP 1 299 090 B1

## Description

La présente invention concerne le domaine technique des systèmes galéniques à libération prolongée et contrôlée de principes actifs médicamenteux et/ou nutritionnels (**PA**), destinée à une administration par la voie orale.

Plus précisément, l'invention vise une composition pharmaceutique pulvérulente administrable *per os* et comprenant au moins un principe actif **PA** notamment absorbé dans les parties hautes du tractus gastro intestinal et des excipients aptes à lui conférer des propriétés d'absorption prolongée du **PA.**

La voie orale est la voie d'administration la plus commode et la plus communément utilisée pour les principes actifs médicamenteux et/ou diététiques. Ces systèmes ont des avantages évidents en termes de facilité d'administration et de tolérance pour les patients.

Il est particulièrement intéressant de chercher à développer des formes galéniques orales assurant la couverture thérapeutique du patient sur un nycthémère (24 heures). Un tel objectif est ambitieux. En effet, la plupart des **PA** administrés per os sont absorbés dans la partie haute du tractus gastro intestinal qui constitue une "fenêtre d'absorption". La durée de passage du **PA** devant cette fenêtre est limitée en temps. Par conséquent, la durée d'absorption est elle-même limitée. Ainsi, il est généralement admis que les temps de résidence des formes orales ingérées sont de l'ordre de 0,5 à 3 heures environ dans l'estomac et de 2 à 4 heures environ dans l'intestin grêle, selon que le sujet se trouve à jeun ou que les formes orales ingérées sont contenues dans un bol alimentaire conséquent. La durée de bio-absorption d'un **PA** administré per os, et dont l'absorption est limitée aux parties hautes du tractus gastrointestinal (TGI), est donc de quelques heures seulement.

A l'accroissement du temps de bio-absorption, qui est l'objectif premier du système galénique de la présente invention, il faut ajouter la condition de maintien de la bio-disponibilité du **PA** à un niveau satisfaisant et suffisant.

La conjonction d'un accroissement du temps de bio-absorption et le maintien de la bio-disponibilité à un niveau satisfaisant et suffisant, est un objectif délicat à atteindre. Une simple augmentation du temps de libération du **PA** au-delà de la durée naturelle du transit dans le TGI ne conduit en général qu'à libérer une partie du **PA** après la fenêtre d'absorption et donc à abaisser la bio-disponibilité du **PA.**

Dans la pratique, le temps de bio-absorption, (Tabs), se déduit du profil de concentration plasmatique(PCP) du **PA :** c'est le temps au bout duquel, le PCP entre en régime d'élimination pure.
La bio-disponibilité est, elle, évaluée classiquement par le rapport de l'aire sous la courbe du PCP à l'aire sous la courbe du PCP d'une forme à libération immédiate de référence.

Pour tenter de résoudre la problématique exposée ci dessus, de nombreuses propositions techniques sont apparues.

Globalement, les propositions techniques antérieures proposent d'accroître le temps de transit du **PA** en proposant trois types de systèmes galéniques, à savoir :

> ➢ les systèmes flottants de faible densité , qui surnagent sur le contenu gastrique liquide , prolongeant ainsi la durée de présence du **PA** dans les parties hautes du TGI ;
> ➢ les systèmes bio-adhésifs qui adhèrent aux muqueuses gastriques et/ou intestinales ;
> ➢ les systèmes gonflants qui augmentent de volume une fois qu'ils sont en contact avec les liquides gastriques, jusqu'à atteindre des dimensions telles qu'ils ne peuvent pas franchir le pylore et sont donc retenus dans l'estomac.

Certains des systèmes connus peuvent combiner deux ou trois de ces fonctionnalités de flottaison, de bio-adhésion et de gonflement.

La demande de brevet PCT **WO-99/47128** propose une revue de l'art antérieur relatif à ces trois types d'approche pour la mise au point de formes galéniques gastrorétentives, ayant des propriétés de libération prolongée et contrôlée du **PA.**
Cette demande PCT WO-99/47128 propose une forme galénique orale adaptée à des principes actifs dotés d'une forte solubilité dans l'eau et présentant une fenêtre d'absorption limitée à la partie haute du tractus gastro-intestinal (Metformine). Pour ces **PA** de forte solubilité, cette forme galénique prétend résoudre le problème consistant à assurer simultanément :

i) une libération prolongée sans "burst" initial, et
ii) un temps de résidence gastrique prolongé.

Ce système galénique à libération contrôlée de **PA** est biphasique. Il comprend:

> ➢ une phase interne particulaire formée de granules individualisés chargés en **PA.** La particularité de ces granules est d'être *non enrobés* et de comporter un ou plusieurs excipients qui peuvent être :

o un polymère hydrophobe : copolymère de l'acide (méth)acrylique (EUDRAGIT®), éthylcellulose,
o et/ou un polymère hydrophile : carboxyméthylcellulose de sodium ou alginate de sodium,
o et/ou d'autres composés hydrophobes : cires, alcools gras, esters d'acide gras,

➢ et une phase continue solide externe dans laquelle sont noyées les particules de la phase interne, cette phase solide externe continue comportant :

o un ou plusieurs polymères hydrophiles : [hydroxypropylméthylcellulose - HPMC- (de viscosité 5 cps et $1.10^5$ cps), cellulose microcristalline],
o et/ou un ou plusieurs polymères hydrophobes,
o et/ou un ou plusieurs autres composés hydrophobes (cires, alcools gras, esters d'acide gras).

Ce système galénique est, de préférence, sous forme de comprimé. Il est présenté comme ayant un temps de résidence accru dans la partie haute du tractus gastro-intestinal (estomac/intestin grêle) par effet d'augmentation de taille, sans toutefois atteindre une limite supérieure conduisant à l'occlusion.
Un inconvénient de cette forme galénique est qu'elle présente un temps de résidence gastrique variable, à la différence d'une forme galénique microparticulaire, dont le temps de résidence est pondéré par le grand nombre de particules.
Par ailleurs, il est vraisemblable que ce système galénique selon le WO-99/47128 (de préférence un comprimé) ait une faible tenue mécanique en milieu gastrique. Dans une telle hypothèse la libération du PA ne serait plus contrôlée.
La demande de brevet PCT **WO-99/47125** propose une forme galénique dont l'application est limitée aux antihyper-glycémiants de très haute solubilité et plus particulièrement la metformine. Cette forme permet d'obtenir une couverture thérapeutique sur 24 heures après administration orale à l'état nourri. Elle est constituée d'un comprimé macroscopique entouré d'une membrane perméable à l'eau mais non au **PA**. Le coeur de l'invention est la mise au point d'un comprimé libérant le **PA** par effet osmotique. Le contrôle de la libération du **PA** est obtenu en ajustant la pression osmotique par ajout d'un polymère augmentant l'afflux d'eau et en ajustant le débit de sortie du **PA** en aménageant un orifice dans la membrane semi perméable. La bio-disponibilité est maintenue en ajoutant au comprimé un promoteur d'absorption tel qu'un sel biliaire.
Le principal inconvénient réside dans la présence de ce promoteur d'absorption qui peut fragiliser la paroi intestinale et peut, en administration prolongée, avoir des effets secondaires indésirables.
Un autre inconvénient est que cette forme "comprimé" présente un temps de résidence gastrique variable, à la différence d'une forme galénique microparticulaire, dont le temps de résidence est pondéré par le grand nombre de particules.
Le brevet américain **N° 5,472,704** propose un système à libération contrôlée maintenant la bio-disponibilité du **PA** par accroissement du temps de résidence de la forme galénique devant la fenêtre d'absorption du **PA** dans le (TGI). Ce système galénique bio-adhésif est composé d'une pluralité de particules individualisés dont la plus grande dimension est au maximum de 2 500 $\mu$m et en pratique de 300-600 $\mu$m Chaque particule peut comprendre une partie bio-adhésive formée d'un copolymère acrylate et d'hydroxypropylméthylcellulose (HPMC) et une partie discontinue comportant des granules de **PA** enrobés (125-600 $\mu$m) dans lesquels le **PA** est associé à un excipient actif au regard de la libération prolongée et contrôlée dudit **PA,** cet excipient n'ayant pas de propriétés bio-adhésives (l'huile de ricin et/ou le lactose et/ou l'alcool polyvinylique et/ou une huile végétale et/ou un phosphate hydrogéné de calcium...).
La partie bio-adhésive, comprend par exemple :

➢ des dérivés de la cellulose, ou un mélange de copolymère acrylique (CARBOPOL) et d'HPMC,
➢ des agents hydrophobes tels que des sels d'acide stéarique, des huiles végétales hydrogénées, des polyéthylèneglycols, du talc...,
➢ ainsi que des agents de désintégration du type polyvinylpyrrolidone, amidon fonctionnalisé par des groupements méthyle et carboxylate de sodium, amidon, acide alginique, carboxyméthylcellulose de calcium, gomme guar, silice, alginate de sodium, gélatine, pectine...

Les granules de PA enrobé de diamètre 125-600 $\mu$m sont mélangés avec les excipients destinés à former la partie bio-adhésive. Ce mélange est ensuite mis sous forme de comprimés, qui sont ensuite broyés et tamisés, de manière à obtenir une poudre de granules 300-600 $\mu$m.
Le but visé par le système galénique selon l'US N°5,472,704 est de mettre au point un système de bio-adhésif. Or, comme enseigné dans le WO-99/47128 discuté ci-dessus, les systèmes bio-adhésifs n'ont pas démontré leur aptitude à augmenter le temps de séjour dans les parties hautes du TGI. Par conséquent, rien dans ce brevet américain N°5,472,704 ne permet de supposer que le système galénique soit apte à accroître le temps de bio-absorption du **PA.**
On connaît également un autre type de système galénique, constitué par une multiplicité de particules ou microcapsules portant chacune du **PA** enrobé d'une couche de pelliculage à base d'éthylcellulose, de polyvinylpyrrolidone, de stéarate de magnésium et d'huile de ricin, par exemple. Un tel système galénique est divulgué dans la demande PCT **WO-96/11675.** Ces inicrocapsules-réservoir tirent de leur multiplicité un avantage, qui est un temps de vidange gastrique plus régulier et reproductible. De plus, leur taille comprise entre 50 et 1 000 μm ainsi que les caractéristiques de leur enrobage permet d'accroître leur temps de transit dans l'intestin grêle (entre 8 et 24 heures) et , par suite , de maintenir l'absorption du **PA** pendant tout ou partie de ce temps de séjour dans l'intestin grêle.

Comme cela sera démontré clairement ci après, le système galénique multiparticulaire selon le WO 96/11675 est perfectible en ce qui concerne la durée d'absorption et la bio-disponibilité de principes actifs ayant une haute solubilité dans l'eau et absorbés dans la partie haute du TGI, comme par exemple la metformine.

Dans un tel état de la technique, l'un des objectifs essentiels de la présente invention est de fournir un nouveau système galénique perfectionné, pour l'administration orale de principes actifs **PA** en particulier de **PA** ayant une haute solubilité dans l'eau et absorbés dans la partie haute du TGI, ce système devant permettre d'obtenir une couverture thérapeutique performante sur 24 heures.

Un autre objectif essentiel de l'invention est de fournir un système galénique gastrorétentif, pour l'administration par voie orale d'un **PA** ayant une haute solubilité dans l'eau et absorbés dans la partie haute du TGI, ce système présentant une durée de bio-absorption accrue, tout en maintenant la bio-disponibilité du **PA** à un niveau suffisant et satisfaisant.

Un autre objectif essentiel de l'invention est de fournir une composition galénique orale, pour l'administration d'un **PA** ayant une haute solubilité dans l'eau, dont le profil de libération in vitro du **PA** ait une forme sigmoïdale.

Un autre objectif essentiel de l'invention est de fournir une composition galénique orale, de type une dose par 24 heures, qui soit efficace sur le plan thérapeutique, qui soit tolérable par le patient, qui soit économique, qui soit facile à fabriquer et dans laquelle on a recours à une combinaison d'excipients pharmaceutiques classiques et inoffensifs.

Un autre objectif essentiel de l'invention est de fournir un système galénique du type de celui évoqué ci-dessus se présentant sous forme de gélules.

Un autre objectif essentiel de l'invention est de proposer l'utilisation du système ou de la composition galénique orale susmentionnée pour la préparation d'un médicament, en particulier d'un médicament, dont le principe actif est très soluble dans l'eau et plus particulièrement encore, dont le **PA** est un anti-diabétique tel que la metformine.

Ces objectifs, parmi d'autres, sont atteints par la présente invention qui concerne, tout d'abord, la composition pharmaceutique orale comprenant au moins un principe actif (**PA**) et des excipients aptes à conférer à cette composition des propriétés de libération contrôlée et d'absorption prolongée du **PA** dans le tractus gastro-intestinal, cette composition étant du type de celles comportant :

> ➢ d'une part, une pluralité de particules individualisées et enrobées comprenant du **PA** et des excipients,
> ➢ et, d'autre part, une phase continue externe d'excipients dans laquelle est dispersée cette pluralité de particules individualisées et enrobées,

caractérisée en ce que :

> - **a -** elle comprend deux systèmes de libération contrôlée du **PA** associés en série, à savoir : les particules individualisées et enrobées, d'une part, et la phase continue externe, d'autre part ;
> - **b -** Les particules individualisées et enrobées de **PA** sont des microcapsules ayant les caractéristiques suivantes :

> (i) leur pellicule d'enrobage a la composition ci-après :

> **1 -** au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, l'éthylcellulose et/ou l'acétate de cellulose étant particulièrement préférés;

> **2 -** au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, le polyacrylamide et/ou la polyvinylpyrrolidone étant particulièrement préférés;

> **3 -** au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine, l'huile de ricin étant particulèrement préférée ;

> **4 -** et éventuellement au moins un agent tensioactif et/ou lubrifiant, présent à raison de 2 à 20, de préférence de 4 à 15 % en poids sur sec par rapport à la masse totale de la composition d'enrobage et choisi parmi les tensioactifs anioniques, de préférence les sels alcalins ou alcalinoterreux des acides gras, l'acide stéarique et/ou oléique étant préférés, et/ou parmi les tensioactifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium

ou de zinc, ou comme le stéarylfumarate, de préférence de sodium, et/ou le béhénate de glycérol; ledit agent pouvant comprendre un seul ou un mélange des susdits produits ;

(ii) elles possèdent une granulométrie comprise entre 50 et 1 000 μm de préférence entre 100 et 750 μm, et, plus préférentiellement encore, entre 200 et 500 μm;

- c - La phase continue d'excipients fonctionnels comprend :

(i) au moins un polymère hydrophile polyélectrolyte (PPE), apte à gélifier et/ou réticuler, de préférence un polymère acrylique ou cellulosique ou un polysaccharide, et plus préférentiellement encore un alginate ;

(ii) au moins un polymère hydrophile neutre (PN), de préférence choisi dans le groupe comprenant les celluloses, plus spécialement l'hydroxypropylméthylcellulose (HPMC) ou l'hydroxypropylcellulose (HPC) et leurs dérivés ;

(iii) et optionnellement un auxiliaire (AUX) de gélification/réticulation du polymère PPE, de préférence un composé à base d'un cation de valence ≥ 2 de préférence un composé à base de calcium, et plus préférentiellement encore, l'acétate de calcium ;

- d - le mélange formé des particules individualisées selon -b- et de la phase continue selon -c- supra, forme spontanément en présence d'eau dans un test de dissolution **D,** un solide macroscopique composite comprenant une phase continue externe sous forme de gel dans laquelle est incluse une phase discontinue interne formée des particules de **PA** individualisées et enrobées, ce solide macroscopique composite se formant spontanément en un temps inférieur à 30 minutes et préférentiellement compris entre 1 et 20 minutes.

De manière tout à fait avantageuse, dès avant leur évacuation de la matrice, les microcapsules permettent la libération contrôlée du **PA** et son absorption dans la partie haute du tractus gastro-intestinal.
Par ailleurs, sans vouloir être lié par la théorie, on peut supposer que grâce à sa tenue mécanique initiale *in situ,* ce système galénique permet une libération progressive des microparticules (microcapsules) de phase interne dans l'estomac, au fur et à mesure que la matrice gélifiée est érodée par les liquides gastriques.
Suivant une autre caractéristique de l'invention, cette composition présente une courbe de dissolution in vitro dans un test **D** ayant une allure sigmoïdale définie de la façon suivante :

■ il existe un point **T** de la courbe de dissolution dont la tangente passe par l'origine sans recouper la courbe et dont l'abscisse **t** est telle que :

$$t_T \geq 1\,h$$

■ 20 % du **PA** sont libérés dans un temps **t** ≥ 1,5 h.

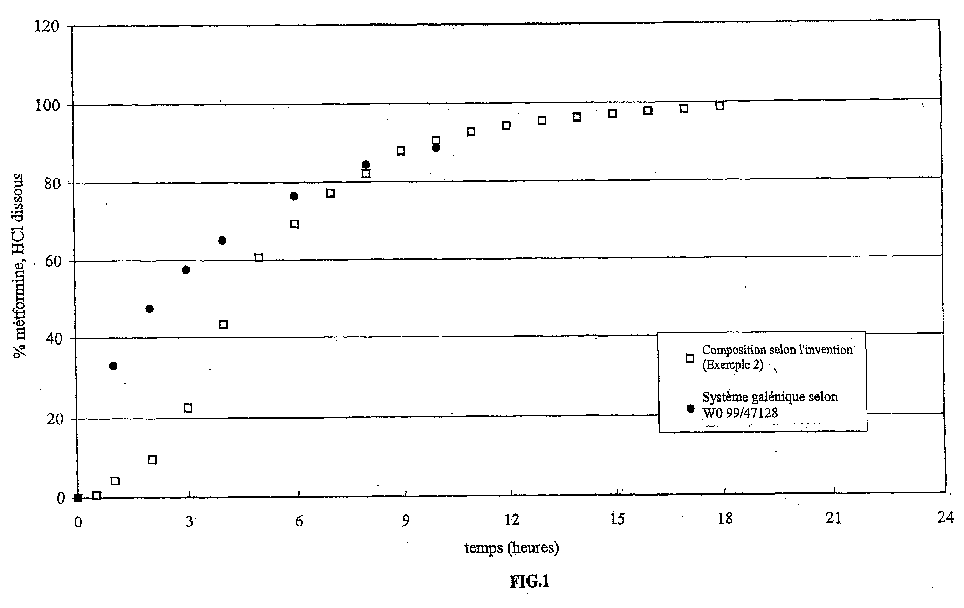

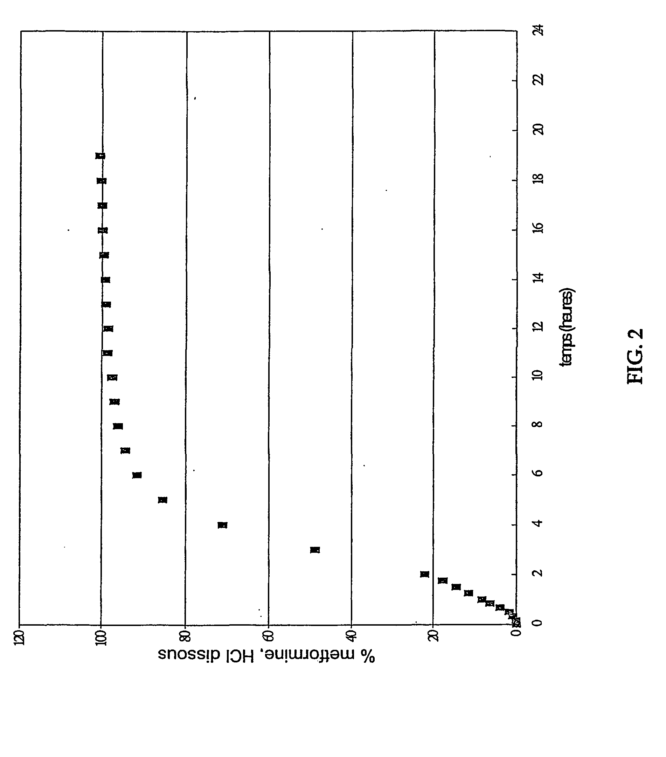

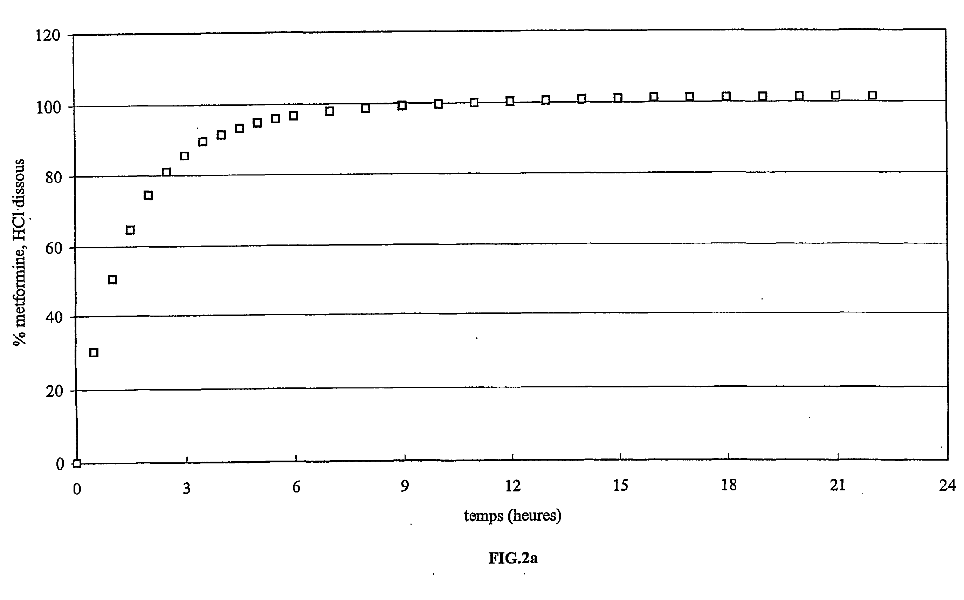

Cette courbe de dissolution in vitro est donnée par un test **D** qui se définit comme suit : *Une gélule contenant la composition galénique orale sous forme de poudre est mise sous agitation à l'aide d'une pale à 100 tours/min, dans un milieu gastrique simulé, à une température de 37°C. Ce milieu gastrique simulé, dont le volume mis en oeuvre est de 1 litre, possède initialement un pH = 1,2. Ce milieu comprend du Nacl à 0,034 mol/l, de l'HCl à 0,063 mol/l et de la pepsine à 3,2g/l. Le pH est progressivement amené à 4,5 par ajout au milieu de pH =1.2, de KH$_2$PO$_4$ (12g) et de NaOH à 35 %.*
Des exemples de courbes de dissolution conformes à l'invention sont représentés sur les figures 1 et 2 annexées.
On remarquera que les courbes de dissolution du **PA** contenu dans les systèmes galéniques selon l'invention, comportent un point **T** dont la tangente passe par l'origine et dont l'abscisse **t$_T$** est ≥ 1 h, de préférence **t$_T$** ≥ 1,5. h et plus préférentiellement encore est : 1 ≤ t$_T$ ≤ 3 h.

En d'autres termes, cela signifie que les courbes de dissolution, dans le test **D,** des compositions selon l'invention, présentent une première partie dans laquelle la libération du **PA** est initialement lente et pour laquelle la concavité est tournée vers le haut, suivie d'une partie pour laquelle la concavité est tournée vers le bas.

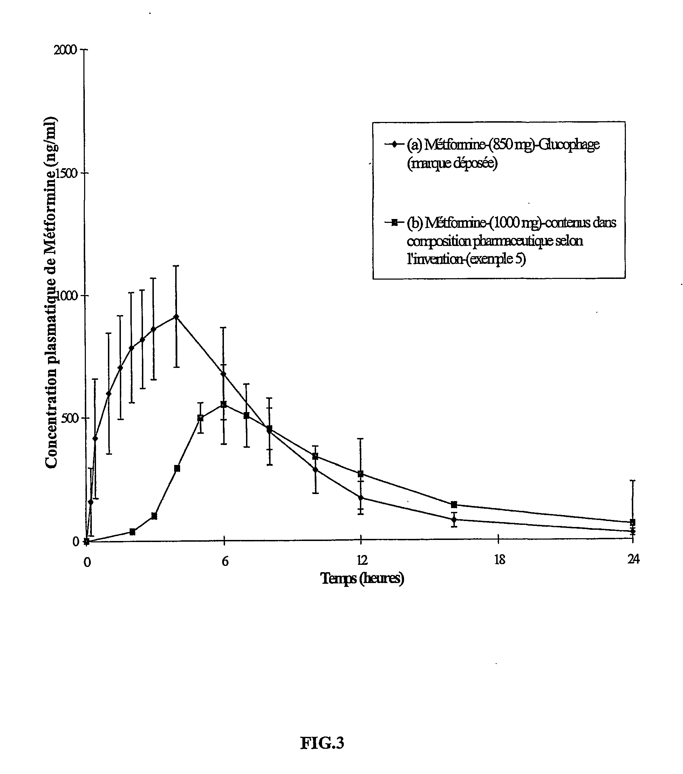

La composition galénique selon l'invention permet d'augmenter la couverture thérapeutique du **PA** par augmentation du $t_{max}$, tout en maintenant la bio-disponibilité à un niveau suffisant et satisfaisant. Les courbes donnant les concentrations plasmatiques en **PA** en fonction du temps suivant la prise, respectivement pour un **PA** (Metformine) à libération immédiate et pour ce même **PA,** dans une composition galénique selon l'invention, sont montrées sur la figure 3. L'augmentation du $t_{max}$ obtenue grâce à la formulation selon l'invention est manifeste.

Suivant une caractéristique préférée de l'invention, la composition de la pellicule d'enrobage des particules individuelles de **PA** est la suivante :

> 1 - 60 à 80 % poids de P1 = éthylcellulose
> 2 - 5 à 10 % poids de P2 = PVP
> 3 - 5 à 10 % poids de plastifiant = huile de ricin
> 4 - 2 à 8 % poids de lubrifiant/ tensioactif = stéarate de Magnésium.

S'agissant de la phase continue externe ou matrice, il est préférable que sa composition soit la suivante :

> i - 60 à 90 % en poids, de préférence de 70 à 90 % en poids, de polymère hydrophile polyélectrolyte (PPE) gélifiant/réticulant, avantageusement d'alginate ;
> ii - 5 à 40 % en poids, de préférence de 10 à 30 % en poids, de polymère cellulosique (PN) hydrophile neutre, avantageusement d'HPMC;
> iii - 1 à 5 en poids, de préférence de 2 à 4 % en poids, d'un auxiliaire de gélification/réticulation (AUX), avantageusement l'acétate de calcium.

La viscosité η peut éventuellement être un critère de sélection des polymères PPE et PN.
Cette viscosité η est par convention une viscosité mesurée à 25 °C pour une solution de polymère dont le titre peut varier, par exemple : 1,25 ou 2 %. La méthodologie utilisée est celle fixée par la pharmacopée US, à savoir l'USP 2208.

Ainsi, s'agissant du polymère PPE et plus spécialement encore de l'alginate de sodium, on retient les produits dont la viscosité η est comprise :

> entre 300 et 1000 mPa.s,
> de préférence pour une solution à 1,25 % dans l'eau. entre 600 et 900 mPa.s,

Concernant le polymère hydrophile neutre PN, sa viscosité η est ≥ 10 000 mPa.s,

> de préférence : 50 000 mPa.s ≤ η ≤ 150 000 mPa.s,
> et plus préférentiellement encore : pour une solution à 2 % dans l'eau. 80 000 mPa.s ≤ η ≤ 120 000 mPa.s

A titre d'autres exemples de polymères (PPE) gélifiant, on peut citer les polyacides acryliques, les gommes xanthane, la carboxyméthylcellulose.

L'HPMC n'est pas le seul polymère neutre hydrophile (PN) à pouvoir convenir dans le cadre de l'invention. A titre alternatif, on pourrait également employer l'hydroxypropylcellulose (HPC).

Les auxiliaires de gélification sont spécifiques des polymères sur lesquels ils exercent leur action. A titre d'illustration, les sels de barium, de strontium, de cuivre, de nickel, de zinc ou de manganèse réticulent l'alginate, conduisant à la formation d'un gel.

Pour poursuivre sur des considérations pondérales, il est à noter que la composition selon l'invention a pour caractéristique avantageuse de comprendre :

> de 50 à 80 % en poids, de préférence de 60 à 70 % en poids, de phase continue externe,
> et 50 à 20 % en poids, de préférence de 40 à 30 % en poids, de particules de **PA** et d'excipients individualisées et enrobées.

Sans que cela ne soit limitatif, la composition orale selon l'invention est, de préférence, pulvérulente.

La composition orale selon l'invention est une forme pulvérulente contenue dans une gélule qui, dans un test **D** de dissolution in vitro, forme spontanément en présence d'eau un solide cohésif formé d'une matrice gélifiée à base de la phase externe continue, et incluant les particules individualisées de **PA** enrobé d'excipients. Ce solide cohésif se forme en moins de 30 minutes et de préférence entre 1 et 20 minutes. Il maintient sa cohésion dans le test **D** durant 3 heures au moins, assurant ainsi d'une part la formation d'un objet d'une taille telle que ne puisse pas être expulsée de l'estomac pendant le temps de la digestion où le pylore est en position fermée. Cependant durant cette phase, la libération du **PA** s'effectue dans le système grâce à la pression osmotique exercée par le principe actif.

Au bout de quelques heures l'objet se délite libérant ainsi les microparticules qui peuvent alors migrer vers le petit intestin où elles vont continuer à libérer le **PA,** accroissant ainsi le temps d'absorption du **PA** dans l'organisme.

Selon une variante, ce mélange pulvérulent pourrait être mis sous la forme de comprimés aptes à se transformer dans le tractus gastro-intestinal en un système comportant une matrice gélifiée à base de la phase externe continue incluant les particules de **PA** et d'excipients individualisées et enrobées.

Comme il a été vu ci-dessus, les excipients sélectionnés et la manière dont ils sont agencés dans le système galénique, sont des caractéristiques essentielles de l'invention. Mais les fonctionnalités de ces excipients s'expriment d'autant mieux que le **PA** appartient à au moins l'une des familles de substances actives suivantes :

antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques;

ce **PA** étant de préférence choisi parmi les composés suivants :

metformine, pentoxyfylline, prazosine, diltiazem, ketoprofen, métoprolol, captopril, aténolol, salbutamol, ranitidine, quinidine, périndopril, morphine, vérapamil et leurs mélanges.

Les principes actifs également concernés par l'invention pourraient être des suppléments nutritionnels et/ou diététiques ou leurs mélanges, comme par exemple des vitamines, des acides aminés, des antioxydants ou des oligo-éléments ou leurs mélanges.

Eventuellement, les vaccins pourraient constituer d'autres **PA** médicamenteux.

Sur le plan quantitatif, le **PA** est présent à raison d'au moins 10 % en poids, de préférence à raison de 15 à 50 % en poids, et plus préférentiellement encore à raison de 20 à 40 % en poids.

Suivant une caractéristique préférée de l'invention, le système galénique qu'elle concerne comprend la composition telle que définie ci-dessus, cette composition étant contenue -de préférence- dans une gélule, par exemple en gélatine, de préférence dans une quantité comprise entre 300 et 1 000 mg, et plus préférentiellement encore entre 400 et 700 mg.

Suivant un autre de ses aspects, l'invention concerne l'utilisation de la composition telle que définie ci-dessus pour la préparation de formes pharmaceutiques ou diététiques, de préférence pulvérulentes et contenues dans des gélules.

S'agissant de la préparation de la composition galénique orale selon l'invention, elle se décompose :

➢ d'une part en l'obtention de la phase interne discontinue de particules de **PA** individualisées et enrobées,
➢ et d'autre part, en l'obtention d'un mélange pulvérulent d'excipients formant la phase externe continue, après hydratation.

Les deux phases externe et interne sont ensuite mélangées et éventuellement transformées en comprimés.

Naturellement, le système galénique selon l'invention pourrait contenir d'autres excipients non toxiques utilisés par l'homme de l'art dans les formes gélules et comprimés. Des conservateurs, des stabilisants, des anti-adhérents, et des agents de masquage de goût, peuvent également être employés.

Concernant la préparation des particules discrètes enrobées formant la phase interne, on se référera à la demande PCT WO-96/11675 qui est intégrée entièrement dans le présent exposé par référence. Plus précisément, on procède à l'enrobage de particule de **PA** par pulvérisation de la composition d'enrobage sur les particules de **PA** mises en mouvement, de préférence par agitation mécanique ou par fluidisation.

Concernant la phase continue externe, il s'agit de procéder à un mélange de poudres, voire à un mélange de poudres et de solutions et à un séchage par tout moyen connu de l'homme de l'art.

Les exemples qui suivent permettront de mieux comprendre l'invention et de saisir tous ses avantages ainsi que les variantes de réalisation envisageables, sans sortir du cadre de l'invention.

## EXEMPLES

## DESCRIPTION DES FIGURES

Les **figures 1** et **2** représentent le profil de dissolution d'un **PA** (metformine) exprimé par le % en poids de **PA** dissous dans le test **D** in vitro en fonction du temps en heures, pour les compositions des exemples 2 et 3, respectivement.

La **figure 2a** représente le profil de dissolution d'un **PA** (metformine) exprimé par le % en poids de **PA** dissous dans le test **D** in vitro en fonction du temps en heures, pour les microparticules selon le WO 96/11675 prises à elles seules et préparées selon la méthodologie décrite au point 1.2 de l'exemple 1.

La **figure 3** représente les profils de concentration plasmatique en metformine après administration per os en dose unique chez le sujet :

(a) : de 850 mg de metformine contenus dans la forme à libération immédiate Glucophage®

(b) : de 1000 mg de metformine contenus dans la forme galénique selon la présente invention.

On se référera à l'exemple 5 pour plus de détails.

## EXEMPLE 1

### 1.1- Produits mis en oeuvre :

a- Principe actif **PA :**

- Metformine/HCl, commercialisée par la Société INTERCHEMICAL.

b - Excipient d'enrobage des particules de phase interne :

- Ethylcellulose caractérisée par un taux d'éthoxyl compris entre 48 et 49.5% et une viscosité comprise entre 6 et 8 mPa.s (CP) fabriquée par la société Dow et commercialisée sous la dénomination ETHOCEL 7
- Stéarate de magnésium commercialisé par la société Ackros
- Polyvinylpyrrolidone fabriquée et commercialisée par la société ISP sous la dénomination PLASDONE K29/32
- Huile de ricin commercialisée par la société Garbit Huileries.

c- Excipient en phase externe :

- Alginate de sodium caractérisé par une viscosité comprise entre 600 et 900 mPa·s (CP) commercialisé par la société Monsanto sous la dénomination KELTONE HVCR
- Hydroxypropylméthylcellulose caractérisée par une viscosité comprise entre 80000 et 120000 mPa.s (CP) commercialisé par la société Colorcon sous la dénomination METHOCEL K 100 M Premium EP
- Acétate de calcium fabriqué par la société Dr. Paul Lohman, qualité poudre USP23

d - Gélule :

gélules vertes opaques de taille 00.fabriquées et commercialisées par Capsugel,

### 1.2 - Méthodologie

1 kg de metformine, HCl tamisée entre 200 et 500$\mu$m, a été pelliculé dans un appareil à lit d'air fluidisé (Niro, precision coater) avec une solution acétone/isopropanol (60/40 (%) (m/m) à 8% (m/m) constituée d'un mélange d'éthocel 7, de plasdone K29/32, d'huile de ricin et de stéarate de magnésium (exemple de composition et de quantité d'enrobage déposée dans les tableaux 1 et 2). Ces particules de metformine pelliculées ont été ensuite mélangées à sec dans un mélangeur cubique, avec un mélange de poudres d'alginate de sodium, d'hydroxypropylméthylcellulose et d'acétate de calcium. Ce mélange a été finalement introduit dans des gélules de taille 00.

La libération de la metformine, HCl a été testée in vitro par le test **D**.

EXEMPLE 2

On prépare une gélule dosée à 142,9 mg de metformine HCl, le taux d'enrobage déposé sur les microparticules de metformine, HCl est de 26%.
Le tableau 1 ci-après regroupe des données quantitatives.

tableau 1 : composition centésimale de l'exemple 2

| Composants | composition centésimale % (m/m) | composition unitaire (mg) |
|---|---|---|
| Metformine, HCl | 25,47 | 142,9mg |
| Ethocel 7 | 6,60 | 37,1mg |
| Stéarate de magnésium | 0,89 | 5,0mg |
| Huile de ricin | 0,72 | 4,0mg |
| Plasdone K 29/32 | 0,72 | 4,0mg |
| Keltone HVCR | 50,91 | 285,6mg |
| Méthocel prémium K 100M | 13,12 | 73,6mg |
| Acétate de calcium | 1,57 | 8,8mg |

La figure 1 montre le profil de dissolution obtenu. On constate sur cette figure 1, qu'elle comprend un point T dont la tangente passe par l'origine et dont l'abscisse T = 5 h 20. Un tel profil de dissolution est révélateur d'une libération prolongée et contrôlée du **PA.** Cela montre également que la forme galénique selon l'invention conserve une intégrité mécanique (masse - dimension - cohésion) pendant une durée relativement longue (au moins 4h). La concavité de la première partie de la courbe (0 - 4 h) est tournée vers le haut : la cinétique de libération est lente et contrôlée.
Cette figure 1 fait également apparaître le profil de dissolution du système galénique selon la demande PCT WO-99/47128.
Il importe de noter la différence d'aspect entre les deux courbes, ce qui correspond à des différences significatives sur le plan du comportement in vivo et donc de l'absorption du **PA.**

EXEMPLE 3

On prépare une gélule dosée à 166,7 mg de metformine, HCl, le taux d'enrobage déposé sur les microparticules de metformine, HCl est de 12%.
Le tableau 2 ci-après présente des données quantitatives.

tableau 2 : composition centésimale de l'exemple 3

| Composants | Composition centésimale % (m/m) | Composition unitaire (mg) |
|---|---|---|
| Metformine, HC1 | 30,31 | 166,7mg |
| Ethocel 7 | 3,06 | 16,8mg |
| Stéarate de magnésium | 0,41 | 2,3mg |
| Huile de ricin | 0,33 | 1,8mg |
| Plasdone K 29/32 | 0,33 | 1,8mg |
| Keltone HVCR | 50,87 | 279,8mg |
| Méthocel prémium K 100M | 13,11 | 72,1mg |
| Acétate de calcium | 1,58 | 8,7mg |

La figure 2 ci-jointe montre le profil de dissolution obtenu. Chaque point de cette courbe correspond à une moyenne obtenue sur 16 gélules.
La courbe de la figure 2 correspondant à la composition selon l'invention, comprend un point T dont la tangente + passe par l'origine et dont l'abscisse $t_T$ = 4 h. Le profil de la figure 2 est de forme sigmoïdale.
Il se distingue nettement du profil de dissolution obtenu avec les microparticules enrobés seules (Fig. 2a) telles qu'obtenues selon la méthodologie décrite au point 1.2 supra de l'exemple 1.
La comparaison des figures 2 et 2a montre également que la forme galénique selon l'invention conserve une intégrité mécanique (masse - dimension - cohésion) pendant une durée relativement longue (au moins 4H). La concavité de la première partie de la courbe (0 - 4 H) est tournée vers le haut.

EXEMPLE 4

*Tenue mécanique en milieu gastrique simulé de la composition selon l'invention conditionnée dans des gélules.*

Conditions de dissolution :

Dissolution à 37°C, agitation avec une pale à 100 tours/min, volume de dissolution = 1 litre.

- pH = 1.2 : ce milieu est constitué de NaCl à 0,034 mol/l, HCl à 0,063 mol/l avec de la pepsine 3,2g/l.
- pH = 4.5 : ajout au milieu de pH = 1.2 de $KH_2PO_4$ (12g) et de NaOH à 35 %.

Les gélules testées présentent la composition décrite dans l'exemple 2 et sont préparées selon le procédé décrit exemple 1.

Le tableau 3 ci-dessous regroupe des données quantitatives :

tableau 3 : données quantitatives de tenue mécanique

|  | L (mm) | 1 (mm) | m (mg) |
|---|---|---|---|
| 1 h à pH =1,2 | 25,7 $\pm$ 0,5 | 10,7 $\pm$ 0,6 | 1501 $\pm$ 68 |
| 2h à pH = 4,5 | 27,7 $\pm$ 1,7 | 12 $\pm$ 1,4 | 2478 $\pm$ 214 |
| 4h à pH = 4,5 | 22,3 $\pm$ 1,7 | 12,3 $\pm$ 1,7 | 1737 $\pm$ 229 |

avec L, longueur du cylindre de gel, 1 diamètre du cylindre de gel, m, la masse du gel (gel $\pm$ eau interne).

Il apparaît qu'au bout de 4h en milieu de pH = 4,5, L et 1 n'ont sensiblement pas variés. Les gels formés à base de la composition galénique selon l'invention ont conservé leur intégrité et leurs dimensions.

EXEMPLE 5

1000mg de metformine répartis en 7 gélules de taille 00 contenant chacune 561 mg de la forme pharmaceutique selon la présente invention ont été administrés à 6 sujets sains après prise d'un repas. La concentration plasmatique en metformine est enregistrée en fonction du temps entre 0 et 36 heures après administration.

Dans cette forme pharmaceutique selon l'invention, les granules de metformine représentent une fraction pondérale de 25.5%, l'enrobage des granules une fraction pondérale de 8.9%, et enfin la phase continue externe une fraction pondérale de 65.6%.

Les compositions sont les suivantes :

*Pour le granulé de metformine :*

| metformine | 100 % | 142.9 mg |
|---|---|---|

*pour l'enrobage :*

| Ethocel 7 | 74% | 37.1 mg |
|---|---|---|
| Stéarate de magnésium | 10 % | 5 mg |
| Plasdone K 29/32 | 8% | 4 mg |
| Huile de ricin | 8% | 4 mg |

*Pour la phase continue :*

| Keltone HVCR | 77.6 % | 285.6 mg |
|---|---|---|
| Methocel K100M | 20.0 % | 73.6 mg |
| acétate de calcium | 2.4 % | 8.8 mg |

Le profil de concentration plasmatique moyen sur les 6 sujets sains est représenté sur la figure 3. A des fins de comparaison, on fait aussi figurer le profil de concentration plasmatique moyen résultant de l'administration à 24 sujets sains après un repas, d'une dose unique de 850 mg de la forme à libération immédiate de metformine, Glucophage®. Ces données sont issues du document FDA (FOI): NDA 20-357. Metformin hydrochloride. LIPHA PHARMACEUTICAL INC.

Les données pharmacocinétiques extraites de ces profils sont reportées dans le tableau 4 ci-dessous :

tableau 4 : données pharmacocinétiques

| Paramètre | formulation selon l'invention | Glucophage® |
|---|---|---|
| dose (mg) | 1000 | 850 |
| Cmax (ng/ml) | 600 | 913 |
| T max (h) | 6 | 4 |
| AUC (ng.h/ml) | 5233 | 7980 |
| T absorption (h) | 10 | 4 |

Il apparaît ainsi clairement que la forme pharmaceutique selon l'invention :

augmente la durée de bioabsorption de façon spectaculaire, augmente également le Tmax et maintient l'AUC à plus de 50% de la valeur correspondant à une forme orale à libération immédiate.

**Revendications**

1. Composition pharmaceutique orale comprenant au moins un principe actif (**PA**) et des excipients aptes à conférer à cette composition des propriétés de libération contrôlée et d'absorption prolongée du **PA dans** le tractus gastro-intestinal, cette composition étant du type de celles comportant :

➢ d'une part, une pluralité de particules individualisées comprenant du **PA** et des excipients ;
➢ et, d'autre part, une phase continue externe d'excipients dans laquelle est dispersée cette pluralité de particules individualisées et enrobées ;

**caractérisée en ce que** :

- **a -** elle comprend deux systèmes de libération contrôlée du **PA** associés en série, à savoir : les particules individualisées et enrobées, d'une part, et la phase continue externe, d'autre part ;
- **b -** les particules individualisées et enrobées de **PA** sont des microcapsules ayant les caractéristiques suivantes :

(i) leur pellicule d'enrobage a la composition ci-après :

**1 -** au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, l'éthylcellulose et/ou l'acétate de cellulose étant particulièrement préférés ;
**2 -** au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, le polyacrylamide et/ou la polyvinylpyrrolidone étant particulièrement préférés ;
**3 -** au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine, l'huile de ricin étant particulèrement préférée ;
**4 -** et éventuellement au moins un agent tensioactif et/ou lubrifiant, présent à raison de 2 à 20, de préférence de 4 à 15 % en poids sur sec par rapport à la masse totale de la composition d'enrobage et choisi parmi les tensioactifs anioniques, de préférence les sels alcalins ou alcalinoterreux des acides gras, l'acide stéarique et/ou oléique étant préférés, et/ou parmi les tensioactifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium ou de zinc, ou comme le stéarylfumarate, de préférence de sodium, et/ou le béhénate de glycérol; ledit agent pouvant comprendre un seul ou un mélange des susdits produits ;

(ii) elles possèdent une granulométrie comprise entre 50 et 1 000 $\mu$m de préférence entre 100 et 750 $\mu$m et, plus préférentiellement encore, entre 200 et 500 $\mu$m ;

**- c -** La phase continue d'excipients fonctionnels comprend :

(i) au moins un polymère hydrophile polyélectrolyte (PPE), apte à gélifier et/ou réticuler, de préférence un polymère acrylique ou cellulosique ou un polysaccharide, et plus préférentiellement encore un alginate ;
(ii) au moins un polymère hydrophile neutre (PN), de préférence choisi dans le groupe comprenant les celluloses, plus spécialement l'hydroxypropylméthylcellulose (HPMC) ou l'hydroxypropyl-cellulose (HPC) et leurs dérivés ;
(iii) et optionnellement un auxiliaire (AUX) de gélification/réticulation du polymère PPE, de préférence un composé à base d' un cation de valence $\geq 2$ de préférence un composé à base de calcium , et plus préférentiellement encore, l'acétate de calcium ;

**- d -** le mélange formé des particules individualisées selon -b- et de la phase continue selon -c- supra, forme spontanément en présence d'eau dans un test de dissolution **D,** un solide macroscopique composite comprenant une phase continue externe sous forme de gel dans laquelle est incluse une phase discontinue interne formée des particules de **PA** individualisées et enrobées, ce solide macroscopique composite se formant spontanément en un temps inférieur à 30 minutes et préférentiellement compris entre 1 et 20 minutes.

2. Composition selon la revendication 1, **caractérisée en ce qu'**elle présente une courbe de dissolution in vitro dans un test **D,** ayant une allure sigmoïdale définie de la façon suivante :

■ il existe un point T de la courbe de dissolution dont la tangente passe par l'origine sans recouper la courbe, et dont l'abscisse $t_T$ est telle que :

$$\mathbf{t_T} \geq 1\,\text{h}$$

■ 20 % du **PA** sont libérés dans un temps $\mathbf{t} \geq 1,5$ h

3. Composition selon la revendication 1 ou 2, **caractérisée en ce que** le polymère PN possède une viscosité $\eta$ à 25 °C $\geq 10\,000$ mPa.s, à une concentration de 2%, et selon les conditions fixées par l'USP 2208.

4. Composition selon l'une quelconque des revendications 1 à 3, **caractérisée en ce que** la composition de la pellicule d'enrobage des particules discrètes de **PA** est la suivante :

➢ 1 - 60 à 80 % poids de P1      = éthylcellulose
➢ 2 - 5 à 10 % poids de P2      = PVP
➢ 3 - 5 à 10 % poids de plastifiant      = huile de ricin
➢ 4 - 2 à 8 % poids de lubrifiant/ tensioactif      = stéarate de Magnésium.

5. Composition selon l'une quelconque des revendications 1 à 3, **caractérisée en ce que** la composition de la phase continue externe est la suivante :

➢ i - 60 à 90 % en poids, de préférence de 70 à 90 % en poids, de polymère hydrophile polyélectrolyte PPE gélifiant/réticulant, avantageusement d'alginate;
➢ ii - 5 à 40 % en poids, de préférence de 10 à30 % en poids, de polymère hydrophile neutre PN, avantageusement d'HPMC;
➢ iii - 1 à 5 en poids, de préférence de 2 à 4 % en poids, d'un auxiliaire de gélification/réticulation AUX, avantageusement l'acétate de calcium.

6. Composition selon l'une quelconque des revendications 1 à 5, **caractérisée en ce qu'**elle comprend :

➢ de 50 à 80 % en poids, de préférence de 60 à 70 % en poids, de phase continue externe,
➢ et 50 à 20 % en poids, de préférence de 40 à 30 % en poids, de particules de **PA** et d'excipients individualisées et enrobées.

**7.** Composition selon l'une quelconque des revendications 1 à 6, **caractérisée en qu'**elle se présente sous la forme d'un mélange pulvérulent propre à se transformer dans le tractus gastro-intestinal en un système comportant une matrice gélifiée à base de la phase externe continue incluant les particules de **PA** et d'excipients, individualisées et enrobées.

**8.** Composition selon la revendication 7, **caractérisée en ce qu'**elle est contenue dans une gélule qui, dans un test **D** de dissolution in vitro, forme spontanément un solide cohésif qui maintient sa cohésion dans le test **D,** pendant au moins 3 h.

**9.** Composition selon l'une quelconque des revendications 1 à 8, **caractérisée en qu'**elle se présente sous la forme d'un comprimé apte à se transformer dans le tractus gastro-intestinal en un système comportant une matrice gélifiée à base de la phase externe continue incluant les particules de **PA**+excipients individualisées et enrobées.

**10.** Composition selon l'une quelconque des revendications 1 à 9, **caractérisée en ce que** le **PA** appartient à au moins l'une des familles de substances actives suivantes :

antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques;
et, de préférence, est choisi parmi les composés suivants :

metformine, pentoxyfylline, prazosine, diltiazem, ketoprofen, métoprolol, captopril, aténolol, salbutamol, ranitidine, quinidine, périndopril, morphine, vérapamil et leurs mélanges.

**11.** Composition selon l'une quelconque des revendications 1 à 10, **caractérisée en ce que** le **PA** est présent à raison d'au moins 10 % en poids, de préférence à raison de 15 à 50 % en poids, et plus préférentiellement encore à raison de 20 à 40 % en poids.

**12.** Système galénique, de préférence sous forme de gélule, **caractérisé en ce qu'**il comprend de la composition selon l'une quelconque des revendications 1 à 10, de préférence dans une quantité comprise entre 300 et 1 000 mg, et plus préférentiellement encore entre 400 et 700 mg.

**13.** Utilisation de la composition selon l'une quelconque des revendications 1 à 12, pour la préparation de formes pharmaceutiques ou diététiques, de préférence pulvérulentes et contenues dans des gélules.

**Claims**

**1.** Oral pharmaceutical composition comprising at least one active principle **(AP)** and excipients capable of giving this composition properties of controlled release and prolonged absorption of the **AP** in the gastrointestinal tract, this composition being of the type containing:

► on the one hand a plurality of individualized particles comprising **AP** and excipients,
► and on the other hand an external continuous phase of excipients in which this plurality of coated individualized particles is dispersed,

**characterized in that**:

- **a -** it comprises two **AP** controlled release systems associated in series, namely: on the one hand the coated individualized particles, and on the other hand the external continuous phase;
- **b -** the coated individualized particles of **AP** are microcapsules having the following characteristics:

(i) their film coating has the following composition:

**1 -** at least one film-forming polymer (P1) insoluble in gastric juices, which is present in an amount of 50 to 90% and preferably of 50 to 80% by dry weight, based on the total weight of the coating composition, and consists of at least one water-insoluble derivative of cellulose, ethyl cellulose and/or cellulose acetate being particularly preferred;

**2 -** at least one nitrogen-containing polymer (P2) which is present in an amount of 2 to 25% and preferably of 5 to 15% by dry weight, based on the total weight of the coating composition, and consists of at least one polyacrylamide and/or poly-N-vinylamide and/or poly-N-vinyllactam, polyacrylamide and/or polyvinylpyrrolidone being particularly preferred;

**3 -** at least one plasticizer which is present in an amount of 2 to 20% and preferably of 4 to 15% by dry weight, based on the total weight of the coating composition, and consists of at least one of the following compounds: glycerol esters, phthalates, citrates, sebacates, cetyl alcohol esters, castor oil, salicylic acid and cutin, castor oil being particularly preferred;

4 - and optionally at least one surfactant and/or lubricant which is present in an amount of 2 to 20% and preferably of 4 to 15% by dry weight, based on the total weight of the coating composition, and is selected from anionic surfactants, preferably alkali metal or alkaline earth metal salts of fatty acids, stearic and/or oleic; acid being preferred, and/or from non-ionic surfactants, preferably polyethoxylated sorbitan esters and/or polyethoxylated castor oil derivatives, and/or from lubricants such as stearates, preferably calcium, magnesium, aluminium or zinc stearates, or such as stearylfumarate, preferably sodium stearylfumarate, and/or glycerol behenate, it being possible for said surfactant and/or lubricant to comprise only one or a mixture of the abovementioned products;

(ii) and they have a particle size of between 50 and 1000 $\mu$m, preferably of between 100 and 750 $\mu$m and particularly preferably of between 200 and 500 $\mu$m;

- **c -** the continuous phase of functional excipients comprises:

(i) at least one polyelectrolyte hydrophilic polymer (PPE) capable of gelling and/or crosslinking, preferably an acrylic or cellulosic polymer or a polysaccharide and particularly preferably an alginate;

(ii) at least one neutral hydrophilic polymer (PN) preferably selected from the group comprising celluloses, more especially hydroxypropyl methyl cellulose (HPMC) or hydroxypropyl cellulose (HPC) and derivatives thereof;

(iii) and optionally an auxiliary (AUX) for gelling/crosslinking the polymer PPE, preferably a compound based on a cation of valency $\geq$2, particularly preferably a calcium-based compound and very particularly preferably calcium acetate;

- **d -** and the mixture formed of the individualized particles according to -b- and the continuous phase according to -c- above spontaneously forms, in the presence of water in a dissolution test **D,** a composite macroscopic solid comprising an external continuous phase in the form of a gel, in which an internal discontinuous phase formed of the coated individualized particles of **AP** is included, this composite macroscopic solid forming spontaneously over a period of less than 30 minutes and preferably of between 1 and 20 minutes.

2. Composition according to claim 1, **characterized in that**, in a test **D,** it has an in vitro dissolution curve whose sigmoid shape is defined as follows:

■ there is a point **T** on the dissolution curve whose tangent passes through the origin without intersecting the curve again, and whose abscissa $t_T$ is such that:

$$t_T \geq 1 \text{ h}$$

■ 20% of the **AP** is released in a time $t \geq 1.5$ h

3. Composition according to claim 1 or 2, **characterized in that** the polymer PN has a viscosity $\eta$ at 25°C of $\geq$10,000 mPa.s at a concentration of 2% and under the conditions set by USP 2208.

4. Composition according to any one of claims 1 to 3, **characterized in that** the composition of the film coating of the discrete particles **of AP** is as follows:

► 1 - 60 to 80% by weight of P 1 = ethyl cellulose
► 2 - 5 to 10% by weight of P2 = PVP
► 3 - 5 to 10% by weight of plasticizer = castor oil
► 4 - 2 to 8% by weight of lubricant/surfactant = magnesium stearate

5. Composition according to any one of claims 1 to 3, **characterized in that** the composition of the external continuous phase is as follows:

► i - 60 to 90% by weight, preferably from 70 to 90% by weight, of gelling/crosslinking polyelectrolyte hydrophilic polymer PPE, advantageously alginate;
► ii - 5 to 40% by weight, preferably from 10 to 30% by weight, of neutral hydrophilic polymer PN, advantageously HPMC;
► iii - 1 to 5% by weight, preferably from 2 to 4% by weight, of gelling/crosslinking auxiliary AUX, advantageously calcium acetate.

6. Composition according to any one of claims 1 to 5, **characterized in that** it comprises:

► from 50 to 80% by weight, preferably from 60 to 70% by weight, of external continuous phase,
► and 50 to 20% by weight, preferably from 40 to 30% by weight, of coated individualized particles of **AP** and excipients.

7. Composition according to any one of claims 1 to 6, **characterized in that** it takes the form of a pulverulent mixture capable of being converted in the gastrointestinal tract to a system comprising a gelled matrix based on the external continuous phase in which the coated individualized particles of **AP** and excipients are included.

8. Composition according to claim 7, **characterized in that** it is contained in a gelatin capsule which, in an in vitro dissolution test **D,** spontaneously forms a cohesive solid that maintains its cohesion in the test **D** for at least 3 h.

9. Composition according to any one of claims 1 to 8, **characterized in that** it takes the form of a tablet capable of being converted in the gastrointestinal tract to a system comprising a gelled matrix based on the external continuous phase in which the coated individualized particles of **AP** + excipients are included.

10. Composition according to any one of claims 1 to 9, **characterized in that** the AP belongs to at least one of the following families of active substances:

antiulcer drugs, antidiabetics, anticoagulants, antithrombics, hypolipidaemics, antiarrhythmics, vasodilators, antiangina drugs, antihypertensives, vasoprotectors, fertility promoters, labour inducers and inhibitors, contraceptives, antibiotics, antifungals, antivirals, anticancer drugs, antiinflammatories, analgesics, antiepileptics, antiparkinsonism drugs, neuroleptics, hypnotics, anxiolytics, psychostimulants, antimigraine drugs, antidepressants, antitussives, antihistamines and antiallergics,
and is preferably selected from the following compounds:

metformin, pentoxifylline, prazosin, diltiazem, ketoprofen, metoprolol, captopril, atenolol, salbutamol, ranitidine, quinidine, perindopril, morphine, verapamil and mixtures thereof.

11. Composition according to any one of claims 1 to 10, **characterized in that** the **AP** is present in an amount of at least 10% by weight, preferably of 15 to 50% by weight and particularly preferably of 20 to 40% by weight.

12. Galenical system, preferably in the form of a gelatin capsule, **characterized in that** it comprises the composition according to any one of claims 1 to 10 in an amount preferably of between 300 and 1000 mg and particularly preferably of between 400 and 700 mg.

13. Use of the composition according to any one of claims 1 to 12 for the preparation of pharmaceutical or dietetic forms that are preferably pulvemlent and contained in gelatin capsules.

**Patentansprüche**

1. Orale pharmazeutische Zusammensetzung, umfassend mindestens ein Wirkprinzip (WP) und Exzipienten, die sich dazu eignen, auf diese Zusammensetzung Eigenschaften einer gesteuerten Freisetzung und verlängerten Absorption des WP im Magen-Darm-Trakt zu übertragen, wobei diese Zusammensetzung so beschaffen ist, dass sie umfasst:

   - einerseits, eine Vielzahl individualisierter Partikel, die das WP und Exzipienten umfassen;
   - andererseits, eine äußere kontinuierliche Phase aus Exzipienten, in denen diese Vielzahl individualisierter und umhüllter Partikel dispergiert ist,

   **dadurch gekennzeichnet, dass** gilt:

   (a) sie umfasst zwei Systeme zur gesteuerten Freisetzung des WP, die nach einander zusammengebracht sind, und zwar: die individualisierten und umhüllten Partikel, einerseits, und die äußere kontinuierliche Phase, andererseits;

   (b) die individualisierten und umhüllten WP-Partikel sind Mikrokapseln mit ,den folgenden charakteristischen Eigenschaften:

   (i) deren Umhüllungsfilm weist die folgende Zusammensetzung auf:

   1) mindestens ein filmbildendes Polymer (P1), das in den Flüssigkeiten des Trakts unlöslich ist, mit 50 bis 90 und vorzugsweise 50 bis 80 Gew.-% auf Trockenbasis, bezogen auf die Gesamtmasse der Umhüllungszusammensetzung, vorhanden und aus mindestens einem wasserlöslichen Cellulosederivat gebildet ist, wobei Ethylcellulose und/oder Celluloselactat besonders bevorzugt sind;
   2) mindestens ein stickstoffhaltiges Polymer (P2), das mit 2 bis 25 und vorzugsweise 5 bis 15 Gew.-% auf Trockenbasis, bezogen auf die Gesamtmasse der Umhüllungszusammensetzung, vorhanden und aus mindestens einem Polyacrylamid und/oder einem Poly-N-vinylamid und/oder einem Poly-N-vinyllactam gebildet ist, wobei Polyacrylamid und/oder Polyvinylpyrrolidon besonders bevorzugt sind;
   3) mindestens ein Plastifiziermittel, das mit 2 bis 20 und vorzugsweise mit 4 bis 15 Gew.-% auf Trockenbasis, bezogen auf die Gesamtmasse der Umhüllungszusammensetzung, vorhanden und aus mindestens einer der folgenden Verbindungen gebildet ist: aus Glycerylestern, Phthalaten, Zitraten, Sebacaten, Estern von Cetylalkohol, Rizinusöl, Salicylsäure und aus Cutinsäuren, wobei das Rizinusöl besonders bevorzugt ist;
   4) und gegebenenfalls mindestens ein oberflächenaktives Mittel und/oder Gleitmittel, die mit 2 bis 20 und vorzugsweise 4 bis 15 Gew.-% auf Trockenbasis, bezogen auf die Gesamtmasse der Umhüllungszusammensetzung, vorhanden und aus anionischen oberflächenaktiven Mitteln, vorzugsweise aus Alkali- oder Erdalkalisalzen von Fettsäuren, wobei Stearin- und/oder Ölsäure bevorzugt sind, und/oder aus nicht-ionischen oberflächenaktiven Mitteln, vorzugsweise aus polyoxethylierten Sorbitanestern und/oder aus polyoxethylierten Rizinusölderivaten, und/oder aus Gleitmitteln wie Stearaten, vorzugsweise von Calcium, Magnesium, Aluminium oder Zink, oder wie Stearylfumarat, vorzugsweise von Natrium, und/oder aus Glycerylbehenat ausgewählt sind, wobei das genannte Mittel ein einzelnes oder eine Mischung der oben genannten Produkte umfassen kann;

   ii) die Partikel besitzen eine Korngröße von 50 bis 1000 $\mu$m, vorzugsweise von 100 bis 750 $\mu$m und noch bevorzugter von 200 bis 500 $\mu$m;

   (c) die kontinuierliche Phase aus funktionalen Exzipienten umfasst:

   i) mindestens ein hydrophiles Polyelektrolyt-Polymer (PPE), das sich zur Gelierung und/oder Vernetzung eignet, vorzugsweise ein Acryl- oder Cellulose- oder Polysaccharidpolymer und noch bevorzugter ein Alginat;
   ii) mindestens ein hydrophiles neutrales Polymer (PN), vorzugsweise ausgewählt aus der Gruppe, umfassend Cellulosen, und ganz besonders Hydroxypropylmethylcellulose (HPMC) oder Hydroxypropylcellulose (HPC) und deren Derivate;
   iii) und gegebenenfalls einen Hilfsstoff (AUX) zur Gelierung/Vernetzung des Polymer PPE, vorzugsweise eine Verbindung auf Basis eines Kations der Wertigkeit $\geq 2$, vorzugsweise eine Verbindung auf Basis von Calcium und noch bevorzugter Calciumacetat;

(d) die Mischung, die aus den individualisierten Partikeln gemäß (b) und aus der kontinuierlichen Phase gemäß obigem (c) gebildet ist, bildet spontan in der Gegenwart von Wasser in einem Auflösungstest D einen festen makroskopischen Komposit, der eine kontinuierliche äußere Phase in Form eines Gels umfasst, worin eine diskontinuierliche innere Phase eingeschlossen ist, die aus den individualisierten und umhüllten WP-Partikeln gebildet ist, wobei sich dieser feste makroskopische Komposit spontan in einer Zeit von weniger als 30 min und vorzugsweise von 1 bis 20 min bildet.

2. Zusammensetzung gemäß Anspruch 1, **dadurch gekennzeichnet, dass** sie eine Kurve einer in vitro-Auflösung in einem Test D ergibt, welche eine S-Form gemäß folgender Definition aufweist:

- es gibt einen Punkt T der Auflösungskurve, deren Tangente durch den Ursprung geht, ohne die Kurve zu schneiden, und für deren Abszisse $t_T$ gilt:

$$t_T \geq 1\ h$$

- 20 % des WP werden in einer Zeit $t \geq 1,5$ h freigesetzt.

3. Zusammensetzung gemäß Anspruch 1 oder 2, **dadurch gekennzeichnet, dass** das Polymer PN eine Viskosität $\eta$ bei 25°C > 10.000 mPa x s bei einer Konzentration von 2 % besitzt, gemäß den durch USP 2208 festgelegten Bedingungen.

4. Zusammensetzung gemäß einem der Ansprüche 1 bis 3, **dadurch gekennzeichnet, dass** die Zusammensetzung des Umhüllungsfilms der diskreten WP-Partikel die folgende ist:

- 1) 60 bis 80 Gew.-% P1 = Ethylcellulose
- 2) 5 bis 10 Gew.-% P2 = PVP
- 3) 5 bis 10 Gew.-% Plastifiziermittel = Rizinusöl
- 4) 2 bis 8 Gew.-% Gleitmittel/oberflächenaktives Mittel = Magnesiumstearat.

5. Zusammensetzung gemäß einem der Ansprüche 1 bis 3, **dadurch gekennzeichnet, dass** die Zusammensetzung der kontinuierlichen äußeren Phase die folgende ist:

- i) 60 bis 90 und vorzugsweise 70 bis 90 Gew.-% hydrophiles Polyelektrolyt-Polymer PPE als Gelier/Vernetzungsmittel, vorteilhaft aus Alginat;
- ii) 5 bis 40 und vorzugsweise 10 bis 30 Gew.-% hydrophiles neutrales Polymer PN, vorteilhaft HPMC;
- iii)1 bis 5 und vorzugsweise 2 bis 4 Gew.-% Gelier/Vernetzungshilfsstoff AUX, vorteilhaft aus Calciumacetat.

6. Zusammensetzung gemäß einem der Ansprüche 1 bis 5, **dadurch gekennzeichnet, dass** sie umfasst:

- 50 bis 80 und vorzugsweise 60 bis 70 Gew.-% kontinuierliche äußere Phase und
- 50 bis 20 und vorzugsweise 40 bis 30 Gew.-% Partikel aus dem WP und den individualisierten umhüllten Exzipienten.

7. Zusammensetzung gemäß einem der Ansprüche 1 bis 6, **dadurch gekennzeichnet, dass** sie in Form einer Pulvermischung vorliegt, um im Magen-Darm-Trakt in ein System überführt zu werden, das eine gelierte Matrix auf Basis der äußeren kontinuierlichen Phase umfasst, die die Partikel aus dem WP und den individualisierten und umhüllten Exzipienten einschließt.

8. Zusammensetzung gemäß Anspruch 7, **dadurch gekennzeichnet, dass** sie in einer Gelatinekapsel enthalten ist, die in einem Test D zur in vitro-Auflösung spontan ein festes Kohäsiv bildet, das seine Kohäsion im Test D während mindestens 3 h beibehält.

9. Zusammensetzung gemäß einem der Ansprüche 1 bis 8, **dadurch gekennzeichnet, dass** sie in Form einer Tablette vorliegt, um im Magen-Darm-Trakt in ein System überführt zu werden, das eine gelierte Matrix auf Basis der äußeren kontinuierlichen Phase umfasst, die die Partikel aus WP + individualisierten umhüllten Exzipienten einschließt.

10. Zusammensetzung gemäß einem der Ansprüche 1 bis 9, **dadurch gekennzeichnet, dass** das WP zu mindestens einer der Familien folgender Wirksubstanzen gehört:

Antimagengeschwürmittel, Antidiabetika, Antikoagulanzien, Antithrombose-Mittel, Hypolipidämie-Mittel, Antiarrhythmika, Vasodilatatoren, Antianginamittel, Antihypertensiva, Vasoprotektoren, Fruchtbarkeitspromotoren, Induktoren und Inhibitoren der Gebärmutteraktivität, Kontrazeptiva, Antibiotika, Antifungika, antivirale Mittel, Mittel gegen Krebs, entzündungshemmende Mittel, Analgetika, Antiepileptika, Antiparkinson-Mittel, Neuroleptika, Hypnotika, Anxiolytika, Psychostimulanzien, Antimigränemittel, Antidepressiva, Antigewebemittel, Antihistaminika oder Antiallergika;
und vorzugsweise ist das WP aus den folgenden Verbindungen ausgewählt:

Methformin, Pentoxyfyllin, Prazosin, Diltiazem, Ketoprofen, Metoprolol, Captopril, Atenolol, Salbutamol, Ranitidin, Chinidin, Perindopril, Morphin, Verapamil und deren Mischungen.

11. Zusammensetzung gemäß einem der Ansprüche 1 bis 10, **dadurch gekennzeichnet, dass** das WP mit mindestens 10, vorzugsweise mit 15 bis 50 und noch bevorzugter mit 20 bis 40 Gew.-% vorhanden ist und vorliegt.

12. Galenisches System, vorzugsweise in Form einer Gelatinekapsel, **dadurch gekennzeichnet, dass** es die Zusammensetzung gemäß einem der Ansprüche 1 bis 10 vorzugsweise in einer Menge von 300 bis 1.000 und noch bevorzugter von 400 bis 700 mg umfasst.

13. Verwendung der Zusammensetzung gemäß einem der Ansprüche 1 bis 12 zur Herstellung pharmazeutischer oder diätetischer Formen, die vorzugsweise pulverförmig vorliegen und in Gelatinekapseln enthalten sind.

18

FIG.1

Legend:
- □ Composition selon l'invention (Exemple 2)
- ● Système galénique selon WO 99/47128

Y-axis: % métformine, HCl dissous
X-axis: temps (heures)

FIG. 2

**FIG.2a**

FIG.3